# EUROPEAN PATENT SPECIFICATION

(11) **EP 1 625 212 B1**
(45) Date of publication and mention of the grant of the patent: **22.08.2007**
(21) Application number: 04733792.8
(22) Date of filing: 19.05.2004
(51) Int. Cl.: C12N 5/06, C12M 3/06, C12M 3/04

(54) **ARTIFICIAL IMMUNE ORGAN**
KÜNSTLICHES IMMUNORGAN
ORGANE D'IMMUNITE ARTIFICIELLE

(30) Priority: 19.05.2003 EP 03011338
(43) Date of publication of application: 15.02.2006
(73) Proprietor: ProBioGen AG, 13086 Berlin (DE)
(72) Inventor: BUSHNAQ-JOSTING, Hikmat, 10439 Berlin (DE); RIEDEL, Marco, 13187 Berlin (DE); MARX, Uwe, 13089 Berlin (DE); GIESE, Christoph, 10439 Berlin (DE)
(74) Representative: Helbing, Jörg
(86) International application number: PCT/EP2004/005412
(87) International publication number: WO 2004/101773

(56) References cited:
- EP-A- 0 690 125
- US-A- 5 677 139
- US-B1- 6 210 959
- US-B1- 6 251 672
- US-B1- 6 479 064

## Description

The invention relates to devices for the faithful modeling of organ functions *in vitro.*

### Background of the invention

The culturing of primary mammalian cells is complicated on principle due to their high sensitivity, relatively slow proliferation, complex differentiating processes and the absolute sterility as a basic condition.

In recent decades, *ex vivo* tissue cultures have been extensively described (Freshney). Cultures with high cell densities or as tissue-like structures require a separation of supply volume and culturing space, e.g., by semipermeable membranes. In addition, this supply principle must realize effective solutions for oxygen supply and other essential nutrients. This has been ensured by special membranes, oxygen transporters in solution etc. In addition, it should be possible to subject the culture to mechanical stresses in order to be able to introduce specific physiological functions and structures (e.g. functional cartilage formation).

In the most effective bioreactor systems, the induction of *de novo* self-organization could be shown *in vitro* in the scientific literature (liver, cartilage, bone, skin etc.).

In the systems described, the cells are usually immobilized in very well supplied cell culturing spaces and can be removed or transplanted as a whole (e.g. encapsulated islet cells) therefrom for therapeutic application. In more complex systems, such as liver substitution systems, the co-cultivation of different cell and tissue types can also be realized. However, in accordance with the respective applications of the culture systems described, none of them provides the possibility of ensuring the continuous regulatable/dimensionable perfusion of the immobilized tissues with at least one mobile tissue type/cell population simultaneously in addition to the *de novo* organization of primary cell and tissue structures in a stationary (non-moving) immobilized state.

In particular, the process of self organization of tissue like structures is induced by cell migration, cell-cell interaction, activation and differentiation due to the local microenvironment. For the formation of multicellular interactions in longterm culture (e.g some weeks) a defined balance between minimal but adequate supply of nutrients, removal of inhibitory metabolites and the maximum support of intrinsic local microgradients must be realized. Cytokines and growthfactors secreted by the immobile cell phase must be held on site to be attractive to the suspended or migrating cells of the mobile phase.

The *in vitro* immunization of human immunocompetent cells ultimately represents the simulation of an organ function *ex vivo* and thus increases the demands on the cell culture to an even higher level. Various cell culture systems are available. In addition to conventional two-dimensional systems in which the cells are growing on a plane surface; mostly on the bottom of the culturing vessel, there are three-dimensional systems for cell culturing. Due to the possibility of cell growth even into the third dimension, the latter offer a tissue-like cell culture which is thus closer to the natural conditions.

Suitable cell and tissue culturing systems are described in the following:

WO 99/43788 describes an *in vitro* model system for viral infection and for immune response which is comprised of a tissue block from tonsil or lymph node supported on a flexible and porous matrix, wherein the tissue block is cultured in a medium whose surface is congruent with the tissue block/matrix interface. The culture system can be used to screen for antiviral drugs, to monitor the course of viral diseases, and to monitor an immune response to antigen stimulation.

WO 89/11529 describes a bioreactor which consists of two chambers, a feed chamber and a discharge chamber, and a cell chamber separated by a selectively permeable ultrafiltration membrane. Within the cell chamber, a biocompatible three-dimensional matrix entraps the animal cells. Due to the presence of this biocompatible matrix, the cell chamber has a gel phase, i.e., the biocompatible matrix and the cells and a liquid phase contain a concentrated solution of the cell product which can be isolated.

US Patent 5,416,022 describes a compact and easily assembled cell culturing device which has at least one cell culturing compartment whose interior represents the cell culturing space.

WO 01/04262 relates to a bioreactor and a method for culturing organic material, especially cells, using a nutrient medium. The culturing of the organic material is effected under a constant flow of nutrients.

DE-C-4230194 and EP-A-0 590 341 describe a reactor for cell culturing which is traversed by two discrete hollow-fiber membrane systems and in which the culturing of the cells is effected on the network formed by the hollow fibers. The reactor is sold under the designation of "Tecnomouse^{®}". The Tecnomouse^{®} bioreactor is a miniaturized perfusion bioreactor which enables three-dimensional cell cultures with continuous circulation of medium and O₂ supply. Five different hollow-fiber modules arranged on top of one another can be run therein simultaneously. The individual modules are supplied with nutrient medium and an air/CO₂ mixture each by the basic device. The supply of nutrient medium is realized by a pump head which enables a variable supply of medium of between 15 and 150 ml/h with five segments. For gas supply, ambient air is passed by means of a membrane pump from the incubator through a gas tower into the respective hollow-fiber module. Through silicone membranes, which limit the corresponding hollow-fiber module on the top and bottom thereof, the gas mixture directly reaches the cell culturing space. The hollow fibers arranged in parallel in narrow meshes form the intercapillary space, ensuring the supply of nutrients and disposal of end products of metabolism. The Cuprophane hollow-fiber membranes employed permit the passage of molecules up to a size of 10 kDa. In the extracapillary space surrounding the hollow fibers, the cells are cultured. Two ports enable inoculation with cells and media as well as cell harvesting. The volume of the extracapillary space of a hollow-fiber module is about 4.3 ml. The Tecnomouse^{®} reactor was first established for the preparation of monoclonal antibodies as an alternative method to animal tests. The development of clean system operation in terms of culture technology and the calculation of volume and size ratios were the subjects of parallel studies, in addition to studies for the characterization of oxygen incorporation (Maier and Nagel, diploma theses TU Berlin 1993; Wiesmann et al., J. Appl. Microbiol. Biotechnol. 41, 531-536 (1994)).

Finally, EP-B-0 584 170 describes a culturing process and a corresponding device in which mammal cells are simultaneously cultivated in several separate culture vessels in a common supply circuit, the supply circuit being separated from the culture vessels by a cell-retaining membrane.

US Patent 5,290,700 also discloses a reactor in which at least two different supply circuits are present.

The reactor according to DE 3409501 and DE 4209501 has two chambers, a cell culturing chamber and a supply chamber, which are separated by a membrane. DE-A-4229334 describes a corresponding jar-like culturing vessel with two chambers.

US Patent 4,242,460 discloses a cell culturing device in a coil form in which the supply circuit is wrapped around the reactor spool as a semipermeable spool. EP-A-0 365 313 describes a cylindrical bioreactor in which the supply is ensured by several pipe systems.

US Patents 4,220,725 and 4,647,539 and DE 3805414 describe a reactor which is traversed by a bundle of semipermeable capillaries.

WO 93/18133 describes a device for treating cell cultures on plate-like antigen cell culture supports which is at least partially gas-permeable and on which a coating of collagen is applied with the cell culture to be cultivated. DE-A-4,322,746 proposes hollow fibers as the corresponding cell culture supports.

EP 0690125 relates to a process for induction culture of a cytotoxic T-lymphozytes having killing activities against tumor cells which comprises the step of co-cultivating a tumor tissue containing said tumor cell and a specific lymphozyte. The tumor tissue may be immobilized.

US Patent 5,677,139 discloses the expansion of hematopoietic T cells. The expression occurs together in culture with stromal cells which may be present as a mono layer.

US Patent 6,479,064 relates to a process for establishing an artificial organ utilizing a three-dimensional scaffold which is covered by a layer of an endothelial tissue layer and is brought in contact with a second culture cell population.

US Patent 6,251,672 relates to a process for cultivating mammalian cells wherein a first mammalian cell is brought into contact with a second mammalian cell which is immobilized on a solid support.

All of the above mentioned three-dimensional culturing methods and devices do not yet approximate the natural system in terms of efficiency. In particular, a controlled growing of the cells and a controlled supply of the cell material to be cultivated with regulatory factors/agents, especially other cell types (in addition to the continuous supply of nutrients), are not ensured.

### Summary of the invention

Surprisingly, it has now been found that the culturing of cells/tissue in a stationary phase by contact with a mobile phase containing cells of a different cell type is essential to the formation of functional tissues, and is of key importance to particular tissue types. Thus, the invention relates to a device for culturing cells and/or tissue comprising at least one culturing space (2) containing matrix(es) (8) suitable for settling cells and/or tissue and at least two fluid delivering means (14, 16; 27, 38) to deliver different fluid to the space (2) or the matrix (8), whereby at least one cell-free supply liquid or supply gas (4) and at least one mobile cell phase (6) is supplied, wherein
(i) said at least one mobile cell phase (6) flows through the matrix (8) in traverse flow; and
(ii) the preferably horizontal matrix (8) is kept in a hollow chamber (20a, 20b) through which said at least one mobile cell phase (6) flows in traverse flow.

The above device is suitable for performing a method for culturing cells and/or tissue comprising the step of culturing cells immobilized in one or more culturing spaces in contact with
(a) at least one cell-free supply liquid or a defined gas mix and
(b) at least one mobile phase containing cells of a cell type different from that of the immobilized cells.

Cells and tissues are obtainable by such a method.

The device according to the invention is suitable, inter alia, for the well-aimed obtaining of human antibodies and human immunocompetent cells against selected antigens (target antigens).

### Brief description of the Figures

Figure 1 shows a first embodiment of the device according to the invention.
Figure 2 shows a second embodiment of the device according to the invention.
Figures 3 and 4 show a detail view and a transverse view, respectively, of the device depicted in Figure 2.
Figure 5 shows panel of surface markers typically used for DC characterization. The bars show parts of cells expressing each marker compared to all cells screened positively for CD45, a pan leucocyte marker before (■) and after (12 h; □) the differentiation process.
Figure 6 shows quantification of phagocytosis by flowcytometry. Leucocytes, e.g. DC can be discriminated from bacteria by nucleic staining (PI on FL-2, A), cells containing FITC-labelled bacteria can be quantified using a quadrant statistic (FL-1, B-D); phagocytosis can be discriminated from adsorption by incubation on ice (C and D).
Figure 7 shows DC forming a dendritic network. T-cells get in close contact to dendrites and are activated for proliferation, T-cells migrate to DC dendrites (A), T-cells get in close contact to DCs (arrow in B), T-cells starts to proliferate in contact to DCs (arrow in C), T-cells are forming floating proliferation clusters (arrow in D).
Figure 8 is a scheme of support lines for a device of the invention.
Figure 9 shows a third embodiment of the device according to the invention.
Figure 10 shows a fourth embodiment of the device according to the invention.

### Detailed description of the invention

According to the present invention, "immobilized cell and tissue culture" means a culture within the device in which the cells are retained within a compartment (culturing space) of the device by adherence to surfaces or by other mechanical or physiological mechanisms.

"Mobile cell phase" or "mobile phase containing cells" means all those cells within the device which are moved through the device in a particular period of time by a directed physical, biophysical or biochemical process, e.g. liquid flows, electromagnetic fields, directed pressures and attractant gradients.

A "cell-free supply liquid" relates to any type of culture medium suitable for cultivation of mammalian cells. Suitable culture medium are in the ambit of the skilled person. A "defined gas mix" according to the invention is adapted to ensure oxygenation of culture volume of 80-100% at 37°C compared to standard air saturation a gas mix is provided containing 20-95% O₂ (v/v). In case of a carbonate buffered culture medium a carbondioxide concentration of 5% (v/v) must be provided to ensure a stable pH at 7.4 in culture volume. Nitrogen is supplemented up to 75% (v/v).

In the method performed with the device according to the invention as described above, a co-culture of suitable cells, e.g., of primary human immunocompetent cells, is ensured over a period of several months. The functionality within the co-culture is ensured by suitable cell populations (cellular components; cell system) which are introduced into a suitable culturing space using a supporting matrix. A continuous supply system (such as that of the device according to embodiment (2) of the invention) is supposed to ensure constant culturing conditions over the entire culturing period. The cell populations employed form functional units by self-organization within the supporting matrix.

The cultured cells and/or tissue (also briefly referred to as "cell system" in the following) are characterized in that cellular structures and immunological functions are mimicked therein which correspond to those of the immunologically active tissues in the form of the germinal centers of a human lymph node or human spleen.

The formation of the cellular organization and microstructure of a germinal center is ensured by a directed concurrence of different cell types. The functions to be mimicked *in vitro* include the directed antigen-induced cell proliferation and antibody expression and the formation of a sustainable immunological memory.

The correct concurrence of antigen-presenting, regulatory and at last antibody expressing cells is necessary for the selective production of an immune response.

The ideal spatial distribution of immobilized cells and the optimal mixing ratio of mobilized cells (e.g. DCs, T lymphocytes, B lymphocytes etc.) according to the method performed with the device of the invention ensures an optimum cell-cell interactions.

In a preferred embodiment, dendritic cells (DCs, APCs) presenting the target antigen are first introduced into the matrix which has been prepared and equilibrated for the culture, and culture is started. Together with the matrix, the adherent DCs represent a so to speak "stationary" phase in the culture system. To this stationary phase, T lymphocytes are suitably added which are infused into the matrix, migrate directionally therein, associate with the DCs in an antigen-specific manner and are thus activated (primed). In a subsequent step or simultaneously, B lymphocytes are infused which in turn associate with the complex of antigen-presenting DCs and correspondingly activated T cells and are also activated in an antigen-specific manner.

Starting from these complexes of APCs and activated T and B lymphocytes, cellular structures are formed which correspond to the *in vivo* formation of a germinal center (GC) or extrafollicular germinal center (efGC).

The *in vitro* efGC and DC are characterized by massive cell proliferation as well as ultimately by antibody expression. They thus increase in cell mass and size. For the initial interaction of the APCs with the mobile migrating lymphocytes, the matrix employed plays a critical role: on the one hand, it ensures the accessibility of the DCs for the lymphocytes, and on the other hand, it stabilizes a system of microgradients of secretory messengers (cytokines) in the immediate surroundings of the cell, which is of key importance to the cell-cell interaction. In addition, the surrounding matrix is increasingly displaced in accordance with the expansion of the germinal centers. At first, IgM antibodies are expressed and presented on the cell surface by the activated B lymphocytes within the germinal centers. Over a period of several days, a change occurs to antibodies of the IgG subclass, which are finally expressed and presented on the cellular surface of plasma cells. Parallel with the change of subclasses, an affinity maturation of the antibody with respect to the target antigen occurs in the germinal center *in vitro* by a somatic permutation. This occurs through a preferential proliferation of the antigen-specific B lymphocytes whose antibodies have a higher affinity for the antigen and therefore have a prolonged cell-cell contact with the APCs as compared with other competing B lymphocytes (also among the T lymphocytes). Thus, their proportion in the total expression of antigen-specific antibodies increases (clonal expansion) and finally dominates in the culture.

The number of specific B and T memory cells (for the induction of a secondary immune response) or naive B and T lymphocytes (for the induction of a primary immune response) is matched to the available APCs in the co-culture. By a suitable ratio of B and T lymphocytes with respect to one another and to the APCs, the suitable proximity of the interacting cells (for a successful migration and association) and the combination of an ideal total cell mass of the co-culture, the directed formation and functionality of the germinal centers in the form of induced antibody production and cell proliferation is ensured.

As antigen-presenting cells (APCs), dendritic cells bear processed antigen on their cell surface and are thus able to activate antigen-specific T4 helper cells (naive or T memory) which are in immediate proximity. The activation causes secretion of cytokines (IL-2, IFNγ) and clonal expansion. The presentation of the antigen is effected through membrane-bound MHC class 2 receptors of the DCs. For a suitable presentation, the antigen is previously bound to microparticles and offered to the APCs for phagocytosis and antigen processing. The addition of the antigen is effected either before or during the co-culturing.

Functional dendritic cells are obtained from blood preparations and differentiated *in vitro.* Lymphocytes are prepared from same or different donors. They can be prepared the same time and cryopreserved during differentiation period of the DCs or prepared at a later date. An autologuous concept is preferred to avoid immunogenic effects between the involved cell populations. For the co-culture, allogenic or autologous T lymphocytes are provided in sufficient amounts and in due time as soon as the differentiation of the DCs and the antigen presentation are completed. Within the scope of cell-cell interaction, the regulatory effects of the T lymphocytes are utilized. For the induction of an existing immune response, these are T memory cells (secondary immune response), while for the selective *de novo* generation of an immune response, naive T lymphocytes are employed (primary immune response). The provision of a sufficient cell mass with a high cell viability and the extension of the lifetime of the prepared cell population are effected by selective T cell expansion.

Also, autologous B lymphocytes are provided in a sufficient amount and in due time for the co-culture. In this case too, memory cells or naive B cells can be employed, depending on whether a secondary or primary immune response is to be induced. The cell mass with high cell viability can be obtained by revitalization of cryopreserved samples at an assigned time.

The T cells, which are predominantly employed for regulatory purposes, can activate suitable specific B cells (naive or B memory) through cell-cell contacts and cytokine secretion and induce them to cytokine secretion and clonal expansion. In addition, the expression of the cell-specific immunoglobulin is either first in the form of IgM (naive; primary immune response) or in the form of IgG (B memory cells, secondary immune response, bystander reaction) which are mobile and migrate by chemotaxis, attracted by the APCs. The cellular interactions are manifested in the form of directed cell migration, the formation of cell-cell contacts, the selective mutually stimulated secretion of cytokines with autocrine and paracrine effects, directed cell proliferation and the antigen-driven affinity maturation of the antibodies formed (antibody arming).

Therefore, important functions of the germinal centers formed *in vitro* consist in a comprehensive directed formation of antigen-specific B cells, the expression of antigen-specific antibodies of various subclasses, the directed change of subclasses and the affinity maturation of antibodies specific for the target antigen.

The supporting extracellular matrix and the culture management additionally ensure the critically necessary cellular tissue-like self-organization and self-conditioning within the co-culture. It must enable the mobility of the cells as well as the adhesion, migration, association and proliferation. In addition, the matrix supports the growth of the germinal centers over culturing periods of several months. For this purpose, optimum diffusion properties for substrates and waste products of the cell metabolism are ensured without disturbing the local microenvironment of cytokine gradients. In addition, a controlled degradation or displacement of the matrix in accordance with the expansion of forming germinal centers is ensured.

In the device according to the invention it is preferred that the matrix of the culturing space has a porosity which enables sufficient flow-through by the mobile phase, migration of the cells within the matrix and the formation of local flow gradients (microenvironment) and preferably has a porosity of more than 30 µm. It is further preferred that the hollow spaces of the matrix have diameters of at least double the diameter of the cells of the mobile phase. Finally it is preferred that the matrix is selected from gels, porous materials, such as open-pore foams, woven fabrics and non-woven fabrics.

The long-term culturing of the germinal centers formed is ensured by continuously supplying the culturing space (e.g., with cell-free supply liquid(s)). The supply system, culturing space and matrix can be sterilized and exhibit sufficient process stability over months for long-term culturing.

The continuous supply ensures a sufficient and consistent supply of substrates and the removal of limiting end products of metabolism. A medium volume circulates through the culture system and is subsequently regenerated (equilibrated) to ideal culture conditions with respect to the oxygen concentration and pH value of the medium. In addition, a portion of the circulating culture medium is continuously changed with a selectively adjustable dilution rate. This ensures a constant supply of the cultured cells with metabolism-relevant substrates and disposal of growth-limiting metabolites even over long culturing periods.

In addition, the physiological state of the culture (substrate consumption, cytokine regression, antibody production) can be monitored by periodically sampling the circulating culture medium.

The culturing space is dimensioned to ensure that a sufficient amount of cells can interact and thus an immune response against any desired antigens can be induced within the scope of an autologous co-culture.

The culturing space itself is filled with a growth-supporting matrix and traversed by microporous membrane surfaces, which ensures the supply of the cells with important substrates and the disposal of critical and desired end products to a sufficient extent without disturbing the microenvironment formed by the cell (gradients of factors having autocrine and paracrine effects).

Sufficient transport of substances from the digestive system through the membrane surfaces into the matrix and finally to the metabolically active cells is controlled by:
1. the material and microstructural appearance of the membrane surfaces;
2. the mutual geometric arrangement of the membrane surfaces;
3. limited diffusion lengths within the culturing space;
4. sufficient transport properties of the matrix in the form of good hydraulic properties and diffusion; and
5. sufficient bioavailability of dissolved substances within the matrix-filled volume.

In particular, the administration and/or removal of oxygen and/or carbon dioxide and/or substrates by cell free supply liquid or gas is provided by microporous membranes of different pore sizes: A hollow fiber with an inner diameter (ID) of 100 to 600 µm, an outer diameter (OD) of 200 to 1000 µm, a wall thickness (WT) of 25 to 200 µm, and a pore size of 0.05 to 0.2. A suitable hollow fiber as diffusion membrane, when using gas is e.g. ID=200 µm, OD=300 µm, polypropylene (PP) and 0.2 mm pore size (Accurel, Membrana GmbH, Germany); and a suitable hollow fiber as filtration membrane when using liquid media (permeable for substrates and metabolites e.g. glucose, lactate) is, e.g. ID=500 µm, OD=700 µm, polyethersulfone (PESF) pore size 0.2 -5 µm (GKSS, Germany).

On the other hand, a constant or periodical perfusion of culture volume by liquid cell free culture media and/or cell suspension is provided by microporous membranes of different pore sizes for perfusion of suspended cells (mobile cell phase), for interaction with stationary cell phase fixed in matrix assisted culture, space for supply of substrates and/or removal of metabolites, for supply of oxygen and/or carbon dioxide and/ or cell free supply liquid or gas, e.g. a planar membrane having a pore size of 10 to 150 µm and a thickness of 0.1 to 2 mm. Suitable membranes are a cell permeable planar teflon membrane of 80 µm pore size (defined isoporous pores) of 1 mm thickness, and a cell permeable, mechanically stabilized teflon grid of 80 µm mesh size (Bohlender, Germany).

The supply system and the culturing space enable a continuous or also periodic sampling of the culture medium and the harvesting of cells and cellular products, such as secretory antibodies.

At the same cell concentration, the percent lymphocyte content in the starting population should be increased. This would mean an approximation to the situation in human blood (M. Classen *et al.,* Urban & Schwarzenberg, Munich, Vienna, Baltimore (1991)) and to the formula proposed by Borrebaeck and Danielsson according to which a ratio of T cells to B cells to accessory cells of 2 : 1 : 0.25 is estimated as an optimum ratio (C.A.K. Borrebaeck, 1989; L. Danielsson et al., Immunology 61 (1987), 51-55). For comparison, the ratio of the mentioned cells in the MNC starting population was 2.7 : 1 : 1 or 0.8 : 0.25 : 0.25 and on the 34^{th} day 7 : 1 : 41 or 0.04 : 0.006 : 0.25. Separation of the cells, especially depletion of the monocyte population, has an unfavorable effect on the course of the culture.

With respect to the composition of the culture medium (and the supply liquid(s)), a higher human serum addition has to prove favorable with respect to medium supplementation. Also by analogy with the results of the described examinations of two-dimensional cultures, the addition of human serum should be about 10-12%. Borrebaeck and Danielsson favor the addition of 10% human serum (C.A.K. Borrebaeck, 1989; L. Danielsson et al., Immunology 61 (1987), 51-55). In the Tecnomouse^{®} bioreactor, 4% HSA was added to the culture medium. Abandonment of FCS as a medium addition seems to be possible. Serum inactivation should be observed strictly in general. Despite the advantageous three-dimensional culturing mode in the bioreactor, an efficient *in vitro* immunization could not be detected in the current stage of development without T or B cell stimulants. In view of the lymphocyte drop, which was disproportionately high in the course, the use of IL-2, e.g., in a concentration of 50 U/ml, and additionally the optional addition of T cell conditioned medium would be advantageous. The employed end concentration of 0.38 µg of HBsAg per ml was within the concentration range also estimated to be favorable by Borrebaeck and Danielsson. Since the cells are not washed by medium in the organ fragment, but are supplied by narrow-meshed medium-bearing hollow fibers in the three-dimensional space, there is no reason for increasing the above mentioned IL-2 or Ag concentration as implied by the increased stimulant demand for lymphocyte activation as described by Ferro and Hoffmann (Ferro et al., Immunobiology 188 (1993) 51-61; Hoffmann et al., J. Immunol. Methods 179 (1995) 37-49).

The method using the device according to the invention enables the following special embodiments:
- long-term operation for multiple immunization, parallel arrangements and dimensions for biomasses;
- process monitoring, for example, by recovering the specific antibodies formed, or cytokine cocktails;
- the recovery of human-type specifically rearranged and hypermutated B lymphocytes/plasma cells and recovery of the individual cell RNA/DNA;
- transfection of a cell line with the antibody gene to produce the monoclonal antibody; and/or
- the recovery of antigen-specific T suppressor/killer cells for cell-substituting autologous therapy.

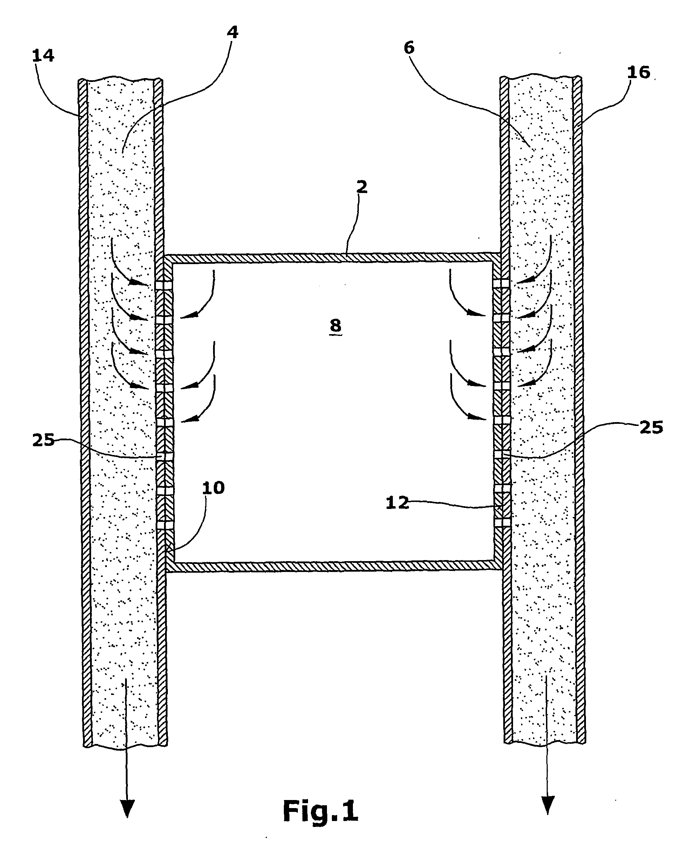

The device according to the invention is suitable, in particular, for performing the method described above. Particular embodiments are shown in Figures 1 to 4 and 8 to 10.

The invention is further illustrated by means of the following Figures and Examples.

### Detailed description of the Figures

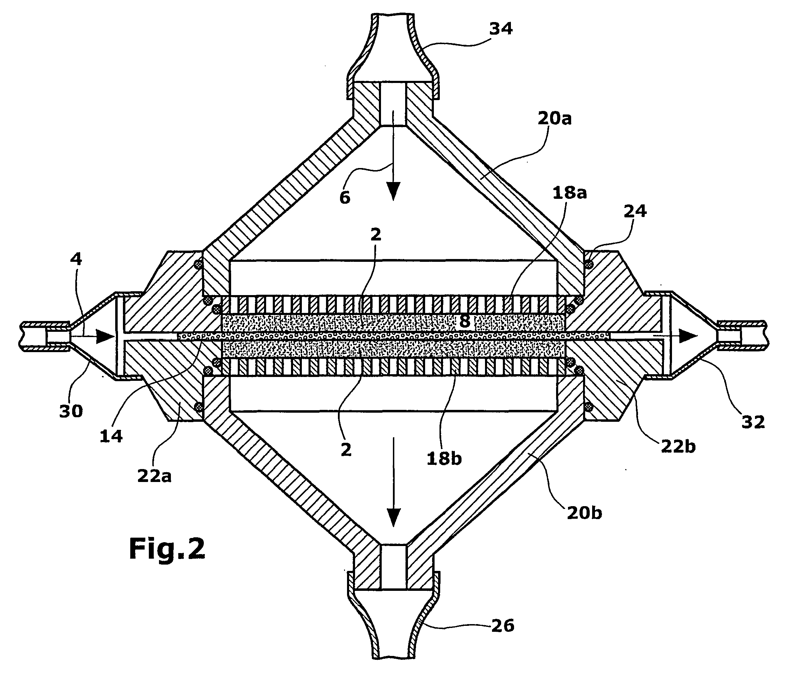

Figures 1 and 2 show alternative embodiments of a device for culturing cells and/or tissue, having a culturing space 2 for settling cells and/or tissue to which different liquid flows 4, 6 can be supplied, preferably continuously, namely at least one cell-free supply liquid 4 and at least one mobile cell phase 6.

In Figure 1, the liquid flows 4, 6 are guided in permeable lines 14, 16 in parallel with a circumferential surface and along this circumferential surface of the culturing space 2.

Due to the permeability of the lines 14, 16, the cell-free supply liquid 4 and the at least one mobile cell phase 6 can diffuse into a matrix 8 contained in the culturing space 2.

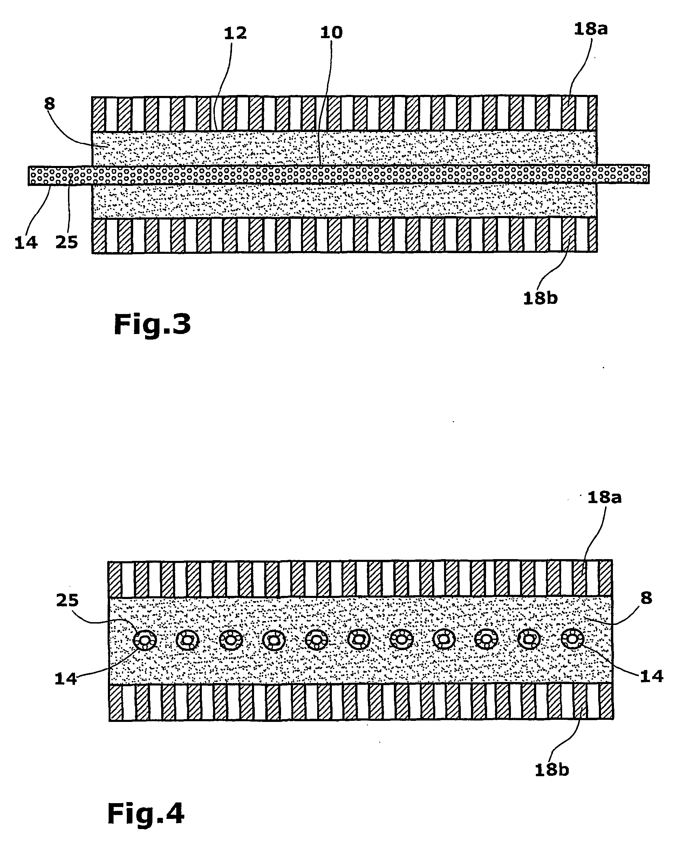

In Figure 1, the material exchange is indicated by arrows which point to outlined pores 25 of the lines 14, 16. The lines preferably consist of capillaries, namely hollow fibers having a diameter of about 50 to 150 µm, preferably about 80 to 120 µm, whose porosity is about 30 to 500 kDa. The pores 25 can be seen best from Figures 3 and 4.

The lines 14, 16 are provided along contact surfaces 10, 12 of the culturing space 2 or matrix 8, Figure 1 also showing passages through the contact surfaces of the culturing space 2 which are representative of the porosity of the matrix.

If several lines 14 for the supply liquids 4 run side by side, preferably in parallel, then their mutual distance is twice to ten times, preferably three to five times, the capillary diameter.

While the lines 14, 16 for the at least one supply liquid 4 and the mobile cell phase 6 are provided along an external lateral surface of the culturing space 2 or matrix 8 in the Example of Figure 1, the Example of Figure 2 provides that the matrix 8 is kept in a hollow chamber 20a, 20b, preferably horizontal, the matrix 8 being flowed through by the at least one mobile cell phase 6 in a traverse flow, while the at least one supply liquid 4 can be supplied through several porous lines 14, especially capillaries, which run through the interior of the matrix 8, preferably in parallel.

The matrix 8 may be supported by a screen 18a, 18b on the bottom side or on both sides. The pore size of the screen 18a, 18b is preferably about 30 to 100 µm.

In the Example of Figure 2, the matrix 8 is a sheet which has a thickness of about 1 to about 15 mm, preferably about 2 to 10 mm. The matrix 8 is held within the hollow chamber formed by two housing halves 20a, 20b which receive the matrix 8 between them with or without supporting devices 18a, 18b. The housing halves have flange parts 22a and 22b which have a port 30 for the supply liquid 4 to be supplied on one side thereof, and, on the other side, a port 32 for discharging the supply liquid 4, which can optionally be recirculated. From the port part 30, the supply liquid 4 is distributed to several lines 14 running preferably midway through the matrix 8 and are arranged in parallel, which can be best seen from Figure 4.

In Figure 2, in a traverse direction with respect to the culturing space 2 or the matrix 8, from top to bottom, a mobile cell phase 6 is supplied, i.e., on the entry side through a port 34. Below the matrix 8, the mobile cell phase can be discharged again through a port 36 and recirculated. The flange parts 22a, 22b are sealed against the housing halves 20a, 20b of the hollow chamber by means of seals 24.

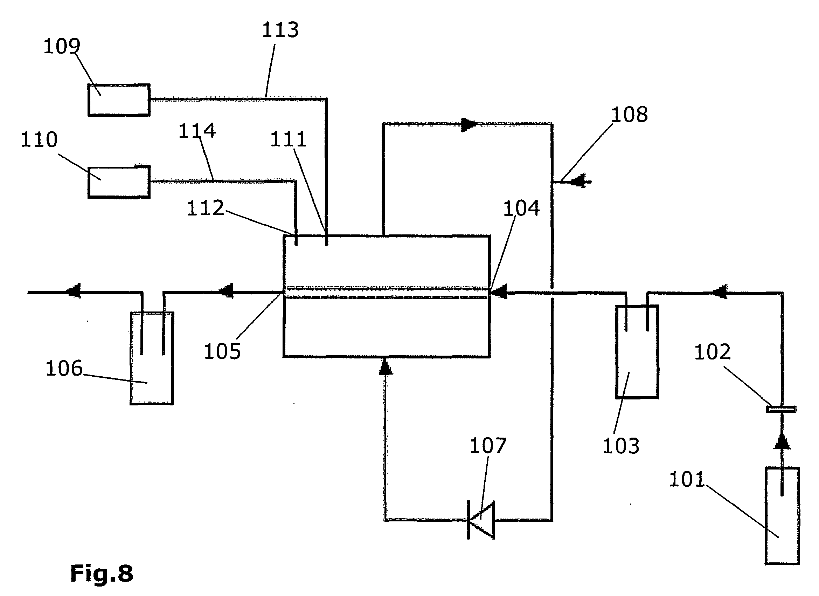

Figure 8 shows a schematic set up for the device of the invention including support lines, controlling and monitoring. A defined prepared gas mix (e. g.: N₂ 75%, O₂ 20%, CO₂ 5%, Linde AG) is applied in a defined flow (e.g. 10-100 cm²/min) to equilibrate a concentration of dissolved oxygen (pO₂ in the culture space of preferably 90% (compared to relative air saturation). Gas is taken from a compressed gas cylinder 101 sterile filtered by a filter 102 and humified in a fritted wash bottle 103. Gas is supplied by the lateral port 104, exhausted at the opposite port 105 and directed into a sterile trap (second fritted wash bottle, 106) to avoid microbial backward contamination.

The circulation of cell suspension and/or cell culture medium for perfusion of the culture module is driven by a peristaltic pumping device (107) with a constant flow of 100 µl/min in a closed loop fluidic system of about 12 ml. A port for injection or probing cell suspension and/or medium is integrated into the circulation loop (108).

Analyzers 109 for pO₂ and analyzers 110 for pH are implemented for monitoring oxygen supply and stabile pH-values (Fibox 3, pH-1 mini, PreSens GmbH). Sterile sensor spots (111, 112) are fixed inside the culture module and read out by fiber optics mounted in the culture module housing. The transparency of the polysulfone used for the culture module housing (thickness at probing port (d = 1 mm) allows the fluorescence optical read out (fluorescence lifetime).

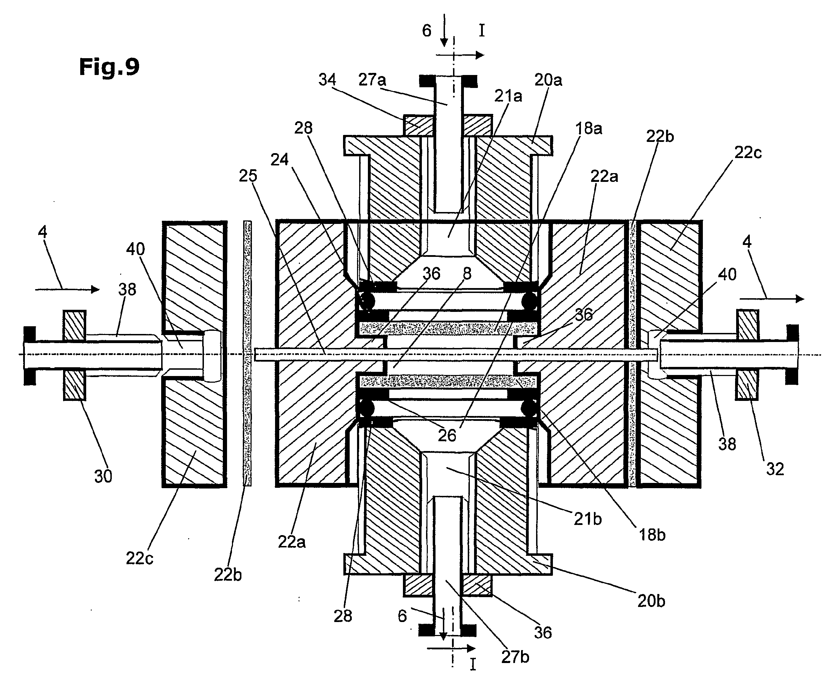

Figure 9 shows an alternative embodiment of device for culturing cells and/or tissue in a sectional view. The device is similar to the one shown in figure 2. Therefore, the same or similar parts are named with the same reference signs.

The main differences are that the housing halfs 20a and 20b are cylindrical so that they can be screwed in the flange part 22a. Each of the housing halfs 20a and 20b has a cylindrical orifice 21a, 21b having a threat to receive a tube 27a, 27b. By connecting the housing halfs 20a, 20b to the flange parts 22a, a good sealing can be achieved using the seals 24, 26, 28. On the other side of the seals, the supporting device 18a, 18b is pressed against a shoulder 36. Between the two supporting devices 18a, 18b the culturing space 2 or matrix 8 is located.

Tubes 38 for the cell-free supply liquid 4 are held in flange parts 22c within orifices 40 where the orifices 40 and the tubes 38 have corresponding threats. Between the flange parts 22a and 22a a third flange part 22b is provided to seal the connection of the two flange parts 22a and 22c.

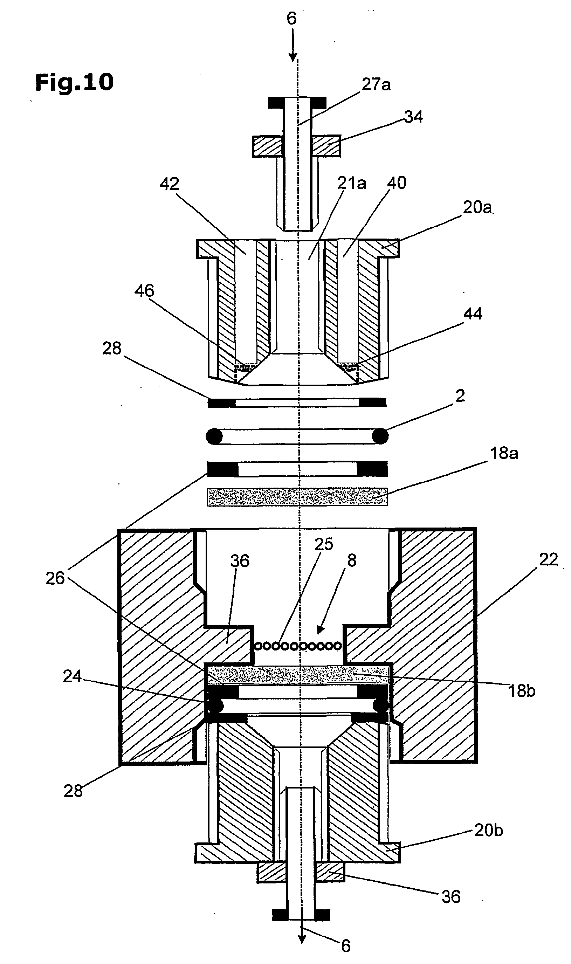

Figure 10 shows a device being similar to the figure 9 in a sectional view along the line I-I in figure 9, whereby the housing half 20a has additional means. To monitor the cells and the media within the matrix 8, two ports 40 and 42 or fiber optics are provided within the housing half 20a. The fiber optics can be connected to a sensor spot 46 for pH or sensor spot 44 for pO₂.

The other part of the device shown in figure 10 is identical to the one of figure 9.

The invention is further described in the following examples.

### Examples

### Materials and Methods

### Chemicals, Commodities and Lab Equiptment:

Blood samples for preparation of primary cells (example 1, 2) (Haema Holding AG, Germany);
Blood samples for preparation of MNCs (example 3, 4) (IKIT, University of Leipzig, Germany);

Bone marrow samples (example 4) (University of Leipzig, Germany);
T-Flasks 75 cm² (Nunclon EasyFlask, Nunc);
T-Flasks 25 cm² (Nunclon EasyFlask, Nunc);
24-well, multiwell plates (Nunclon, Nunc);

Techno Mouse (Integra Biosciences, Germany);
Cell Incubator (CellSafe, Heraeus, Germany);
air supplemented with 5% carbon dioxide, 37°C temperature, humidity 90% rel. hum.;
Flowcytometer FACScalibur (BD Biosciences, Germany);
Cytokine detection for ELISA (Quantikine-Immunoassay, R&D-Systems Bierman, Germany);
Cytokine detection for flowcytometric bead assay (CBA, BD Biosciences, Germany);
Inverted Microscope (Olympus IX-50, Olympus Deutschland, Germany);
Digital Colour CCD-Camera (DP-50, Olympus Deutschland, Germany);
Autoclave (Tecnoclav 120, Fedegari, Integra Biosciences, Germany).

### Sterilization:

Incubation of items in a water steam saturated atmosphere of 121°C temperature and a pressure of 100 kPa over ambient (pressure) for 20 min.

All components of the perfusion bioreactor were sterilized by gamma irradiation (17 kGy) assembled sterilely under a laminar flow device. The inoculation of suspended cells (about 1x10⁶/modules) was done under sterilized conditions as well. The bioreactor setting including the culture module and supporting fluidics is transferred into a temperature controlled incubator (37°C).

### Culture medium:

culture medium RPMI 1640 (Gibco Invitrogen);
fetal calf serum (FCS; HyClone, USA);
cytokines (IL-4, GM-CSF, TNF-α) (AL-Immunotools, Germany);

### Antibodies:

Antibodies for flowcytometry (BD Biosciences, Germany)

### Reagents/Abbreviations:

| | |
|---|---|
| CD1a- CD86 | Cluster of Differentiation defined markers on cell surface established in clinical diagnosis |
| FITC | Fluorescein isothiocanate, fluorochrome for labelling of binding proteins e.g. antibodies |
| PerCP | fluorochrome for labelling of binding proteins e.g. antibodies (different excitation/emission wave length for fluorescence to FITC) |
| PE | Phycoethyrin, fluorochrome for labelling of binding proteins e.g. antibodies(different excitation/emission wave length for fluorescence to FITC or PerCP) |
| T-cells | human T-lymphocytes of peripheral blood preparations |
| B-cells | human B-lymphocytes of peripheral blood preparations |
| APC | Antigen presenting cells are exposing parts of antigenic molecules using the major histocompatibility complex molecules (MHC II) on their cell surface, e.g. dendritic cells (DC) |
| DC | Dendritic cells |
| GC | Germinal centres are histological structure in lymphnode and spleen and play an important role in humoral immune response in man |
| efGC | Extra follicular germinal centres are a special form of germinal centres with reduced and histological structure in lymphnode and spleen and could also be found in non lymphatic organs e.g. synovial seam of extremities |
| IL-2 -IL-12 | Interleukins, high specific messenger substances for communication between leucocytes involved in cellular chemotaxis and cell activation |
| IFN-α, β, γ | Interferon, high specific messenger substances for communication between leucocytes involved in cellular chemotaxis and cell activation |
| GM-CSF | Granulocyte and Macrophage colony stimulation factor, high specific messenger substance for cell activation and differentation |
| FCS | fetal calf serum, biological preparation from blood of unborn calfs |
| DNA / RNA | nucleic acids (desoxyribonucleic acid, ribonucleic acid) specific encoding information molecules of genom and transcriptom in cells |
| | |
| SFM/ SFM2 | cell culture medium for serum free application (Gibco, Invitrogen) |
| | |
| ELISA | Enzyme linked immunosorbent assay, analytical method for specific detection and quantification of peptides and proteins |
| MNC | Mononuclear cells (Leucocyte from peripheral blood preparation) |
| HbsAG | Common hepatitis B virus antigen |
| HLA-DR | Human leucocyte antigen, type DR cellular and tissue antigen factors for recognition of self and non self of the immune system |
| IgM / IgG | Immunoglobulines, antibody molecules of the M or G type |
| | |
| PWM | Pokeweed mitogen, herbal lectine from pokeweed with high immunogenic and mitogenic potency (from Sigma-Aldrich) |
| | |
| LPS | Lipopolysaccharid, parts of bacterial cell wall (E.coli) with high immunogenic and mitogenic potency (from Sigma-Aldrich) |
| | |
| CMV | Cytomegalievirus, latent humanpathogen virus |

### Example 1: Generation and subcultivation of immune competent cells

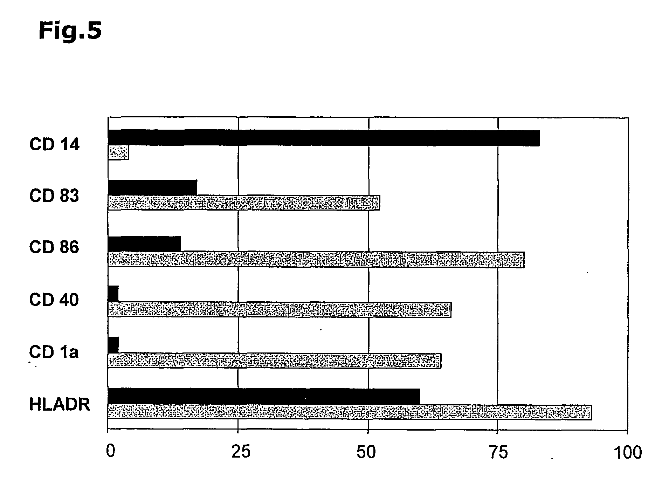

To investigate cellular immune functions *in vitro* B-lymphocytes (B-cells), T-lymphocytes (T-cells) and dendritic cells were subcultivated or generated from human blood samples of a single patient to ensure an autologous concept for further co-cultures composed of different cell populations.
Mononuclear cells (MNC) were separated from whole blood or leukapheresis preparations by densitiy gradient centrifugation. Human immune competent cells were isolated by immune magnetic separations by surface markers in a first step for CD14 and a second step for CD4 or CD19. Additional a depletion of CD25 was applied to remove regulatory cells with suppressive potential.
The isolated subpopulations were cultivated in RPMI 1640 media supplemented with 10% (v/v) fetal calf serum (FCS; HyClone) cultivation.
Monocytes obtained by the CD14-separation were used to differentiate human dendritic cells (DC) by cytokine stimulation. Therefore IL-4 and GM-CSF were supplemented 24 h and 48 h after preparation (each 800 IU/ml for 2x10⁶ cells/ml). Additional TNF-α was added on day 8. DC were characterized by flowcytometry for surface markers and the activity of phagocytosis.
The CD14-negative pool was cultivated in RPMI 1640 media supplemented with 10% (v/v) fetal calf serum and could be cryopreserved by -80°C for three months. The cryoconserving media was composed of RPMI 1640 media, 25% (v/v) fetal calf serum and 10% (v/v) dimethylsulfoxide (DMSO, Sigma-Aldrich).
B-cells and T-cells were obtained using the CD14-negative pool from CD14-(+) separation isolated or after revitalization of cryopreserved samples by separation for CD19 or CD4 respectively.
The generated DC were characterized by flowcytometry for a panel of surface markers (CD-markers) and ability to phagocytosis (Phagotest, BD Biosciences).
After day 12 of differentiation the generated DCs showed typical shifts in the expression of surface markers as described in literature: The monocyte marker CD14 disappeared on the first day of cytokine supplementation (IL-4; GM-CSF). In contrast the marker CD1a escalated. During the process of differentiation CD40, CD83 and CD86 increased significantly. The marker HLA-DR, the major part of the MHC-II complex that is important for antigen presentation was consistently expressed (Fig. 5).

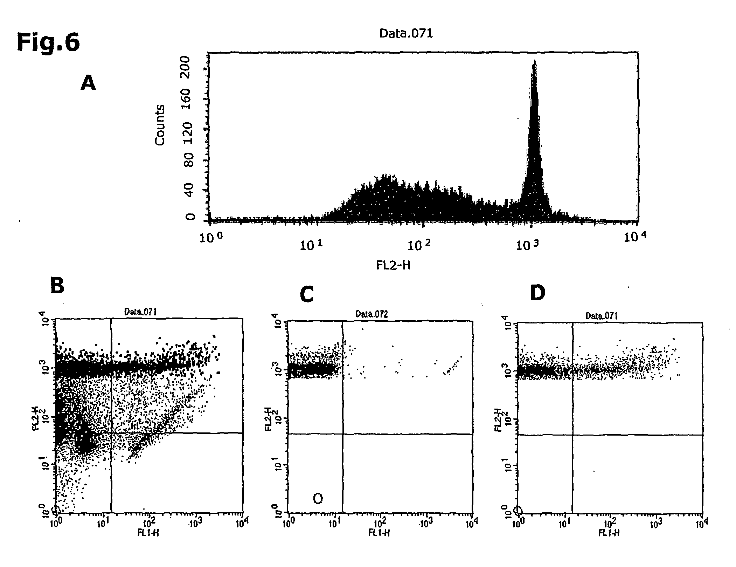

The generated DCs show an enhanced phagocytic activity compared to freshly isolated CD14-positive cells (monocytes/macrophages). Phagocytic activity increased from 6.33 to 16.15 %. Therefore DC were incubated with fluorescent labelled bacteria (*E. coli*-FITC) and fluorescence intensity was measured by flowcytometry. Cells with phagocyted bacteria could be discriminated from free bacteria by additional propidium iodide (PI) staining and from adsorbed bacteria by control incubation on ice (0°C) (Fig. 6).

### Example 2: Spontaneous forming of a complex cellular network by co-cultivation of DC and T-cells

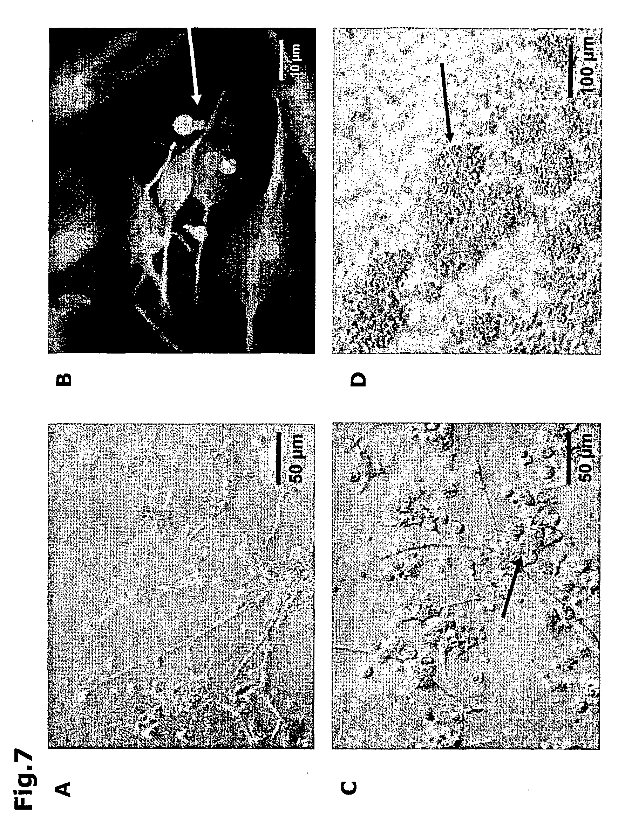

A defined co-culture of DC and T-cells could be established. DC were preincubated in suspension culture with particular antigen (CMV-latex beads, CMV-diagnostic kit, Abbott Diagnostics) for 4 h. Then 10⁵ DC/ml were seeded on multiwell plates (24-well) and stimulated for bystander by pokeweed mitogen (PWM, 1µg/ml). After 24 h of cultivation the DC spread out on the surface of the culture dishes and 10⁵ T-cells were added (unprimed to CMV-Antigen by selection of blood donors without acute or latent CMV infection). The co-culture was monitored for about 14 days. After 6-10 days the DC have formed a complex network of dendrites by a process of self organization (Fig. 7). The formerly suspended T-cells changed to adherence, migrated to the dendritic network and got in close touch to the dendrites. Proliferation of T-cells was initiated and floating proliferation clusters could be observed with loose contact to the culture surface (Fig. 7).

Moreover, the formation of a network in co-cultivated DC and T-cells could be shown in three dimensional cultures also. A hydrogel derived from fibrinogen human blood coagulation system was prepared as described in the manual (Tissucol Immuno Kit, Baxter Bioscience) but using in a ten-fold dilution.
DCs and T-cells were suspended in fibrinogen solution and after induced clotting embedded homogenuously in the gelating matrix to a final concentration of 0.8x10⁶ resp. 4.9x10⁶ cells/ml. The matrix was prepared in disk shaped layers of about 1 mm height. Depending on the concentration of 1:10 to 1:5, diluted by phosphate buffer (PBS) before clotting they had a moderate transparency to opacity.
Embedded cells were stimulated by Mitogen supplementation of 1 µg/ml bacterial lipopolysaccherid (*E. coli*; LPS). Under microscopical control the embedded cells were cultivated for about 10 days.
From day 4 to 6 of the culture period DC form a dendritic network as seen in the 2 dimensional culture by self organization processes. From day 4 to 6 T-cells formerly distributed homogenuously are crowded around the DC by migration and formed proliferation clusters up to the end of culture.

### Example 3: Examination of a three-dimensional long-term culture of human MNCs in a Tecnomouse^{®} bioreactor (Reference Example)

Two different populations from the same preparation of MNCs from a healthy human donor were cultured for 56 and 34 days in a Tecnomouse^{®} bioreactor (description see above). The culture space of about 4.3 ml was inoculated with a starting cell number of 2.4 x 10⁸ (55.8 x 10⁶ cells/ml) and 1.54 x 10⁸ (35.8 x 10⁶ cells/ml). The perfusion system of the bioreactor (intracapillary space) was preincubated by continuous perfusion with culture media IFM from 5 l recirculation jars with a perfusion rate of 75 ml/h for 30 min. The culture space (extracapillary space) was preincubated with culture media SFM1 for 30 min. Cells were suspended in 6 ml of SFM2 and 0.5 ml of HEVAC and transferred into culture space. The medium jars were changed in 14 day intervals. Glutamine was added into intracapillary space in accordance with the nominal concentration in the IFM at 7 day intervals. 2 ml of SFM2 and 0.5 ml of HEVAC were added one time into the intracapillary space on day 34. For supply of the bioreactor the gas mix from a cell cultivation incubator was used (air/CO₂ mixture, 5% CO₂). The gas circulated by 1.8 l/min. For monitoring the metabolic situation, the nutrient supply and for the early recognition of any infections, the glucose and lactate concentrations were measured daily in the cell culture supernatant of the MNC population. Cell counts were performed with the ethidium bromide/acridine orange method. Culture supernatants were analyzed by ELISA for IgM, IgG, anti-lipid A, anti-LPS J5 and anti-HBsAg antibody. The cytokine IL-1b, IL-1ra, IL-2, IL-4, IL-6, IL-8 and TNF-α were analyzed additionally. A comparative FACS analysis of both populations on days 1 and 34 of the culture was supposed to discover any differentiation and proliferation processes occurring during the culture. The cell surface markers CD45, CD2, CD3, CD20, BMA031, CD11a, CD25-F, CD45 were screened to observe shifts in the pattern of differentiation and proliferation markers during culture time of 34 days in MNC-culture.
MNC and lymphocyte population were used and a vitality of about 90% could be detected after 5 weeks of culture. With respect to the percent distribution of immunocompetent subpopulations, the composition of the MNC starting population approximately corresponded to the normal distribution in peripheral human blood as described by Classen (M. Classen et al., Urban & Schwarzenberg Munich, Vienna, Baltimore (1991)). In the first five weeks of the culture, the cell distribution clearly changed in favor of the macrophages and dendritic cells. The detailed evaluation of the FACS analyses shows that proliferations and activation processes took place in both culture variants. The proliferation markers HLA-DR, CD 71 and CD 25 increased in all subpopulations over the first 5 weeks. The only exception were the T suppressor cells in which merely an HLA-DR increase could be seen. Despite of the clear decrease of the lymphocyte counts, a proliferation or activation becomes observable within the lymphocyte population. In both culture variants, an increase of the T cell markers CD2, CD4 and TCRαβ could be detected. A CD8 increase becomes observable only when the median values are considered, so that, to conclude, a push of development of all T cell subpopulations can be derived. This derivation is supported by the reduction of naive T cells (CD45RA) with a simultaneous increase of T memory cells or activated T cells (CD45R0). The continuous detection of CD11a and Lecam1 speaks in favor of the functional expression of adhesion molecules. The antibody concentrations examined in the course by means of ELISA showed that high concentrations of antibodies can be detected even up to week 8 of the culture (see Table 7).

The cytokines IL-8 and IL-1ra could be detected throughout the culture time. The evaluation of the daily determination of glucose and lactate in the recirculating medium (intracapillary space) yielded a glucose and lactate level in the stated concentrations which was stable throughout the course of the culture, which allows to conclude a stable nutrient supply without a critical accumulation of metabolic end products.

The drop of the CD45 RA/R0 ratio and the decrease of the IgM/IgG quotient in the course of time show that a primary and also a secondary immunization seems to be possible already in quite simple examination approaches.

In the daily measurements of the glucose and lactate concentrations over the whole time of the culture course, the glucose content was at least 12.3 mmol/l. The maximum lactate concentration was 1.9 mmol/l.

**Table 1: Results of cell counts in the course of a three-dimensional long-term culture of human MNCs in a Tecnomouse^{®} bioreactor**

| Time | Mononuclear cells (cells/ml) | | | Lymphocytes (cell/ml) | | |
|---|---|---|---|---|---|---|
| | Total | Living | Viability | Total | Living | Viability |
| Start | 55.8 x 10⁶ | n.d. | n.d. | 35.8 x 10⁶ | n.d. | n.d. |
| Day 34 | 334,000 | 302,000 | 90.4% | 182,000 | 167,000 | 91.8% |
| Day 56 | 728,000 | 349,000 | 47.9% | n.d. | n.d. | n.d. |
| n.d. = could not be determined | | | | | | |

**Table 2: Proportion of lymphocytes and macrophages/dendritic cells in the respective total population as determined by FACS analyses:**

| Proportion of total population: | Mononuclear cells, start of culture | Mononuclear cells, day 34 | Lymphocytes, day 34 |
|---|---|---|---|
| Lymphocytes: | 81% | 17% | 8% |
| Macrophages/dendritic cells: | 19% | 83% | 92% |
| Vital macrophages/dendritic cells: | 12% | 20% | 42% |

| | | | |
|---|---|---|---|
| Note: The classification into subpopulations mentioned here was effected by means of the difference in cell deviation in the forward and sideward scatter of the FACS device due to different physical cell properties and through the different fluorescence of the cells after marking with the antibody mixture CD 45-F/CD 14-PE. | | | |

**Table 3: Distribution pattern of different differentiation and proliferation markers on the vital macrophages/dendritic cells in the respective total population**

| Examination of vital macrophages/dendritic cells: | | | | | | | |
|---|---|---|---|---|---|---|---|
| positive antigen detection | Mononuclear cells, start of culture | | Mononuclear cells, day 34 | | Lymphocytes, day 34 | | Antibodies |
| | Fraction | Median | Fraction | Median | Fraction | Median | |
| CD 14 | 77.06% | 542.47 | 88.51% | 523.30 | 88.79% | 588.21 | CD 14-PE |
| HLA-DR | 87.20% | 98.22 | 00.00% | 5048.0 | 99.95% | 4697.59 | HLA-DR-F |
| CD 4 | 95.73% | 7.70 | 64.97% | 7 | 58.81% | 99.10 | CD 4-PerCP |
| CD 71 | 96.81% | 6.85 | 99.32% | 122.98 | 99.86% | 201.69 | CD 71-F |
| CD 25 | 97.99% | 3.22 | 99.88% | 339.82 | 99.99% | 201.69 | CD 25-F |
| | | | | 57.77 | | | |

**Table 4: Distribution pattern of different differentiation and proliferation markers on the lymphocytes in the respective total population**

| Positive antigen detection | Mononuclear cells, start of culture | | Mononuclear cells, day 34 | | Lymphocytes, day 34 | | Antibodies |
|---|---|---|---|---|---|---|---|
| | Fraction | Median | Fraction | Median | Fraction | Median | |
| Control¹ | 0.9% | 4 | 4.9% | 8 | 0.3% | 62 | |
| CD 2 | 74.1% | 15 | 92.6% | 30 | 95.6% | 33 | CD 2-F |
| CD 3 | n.d. | n.d. | 88.6% | 76 | 89.5% | 69 | CD 3-PerCP |
| CD 4 | 44.4% | 36 | 63.2% | 46 | 62.9% | 45 | CD 4-PerCP |
| CD 8 | 20.0% | 155 | 21.6% | 262 | 17.1% | 223 | CD 8-PerCP |
| CD 4/8 ratio | 2.2 | | 2.9 | | 3.7 | | |
| CD 5 | 64.5% | 139 | n.d. | n.d. | n.d. | n.d. | CD 5-PE |
| CD 20 | n.d. | n.d. | 8.8% | 37 | 12.5% | 35 | CD 20- |
| TCR ab | 66.3% | 30 | 87.7% | 33 | 82.6% | 28 | PerCP |
| CD 11a | 95.8% | 47 | 98.1% | 55 | 97.5% | 51 | BMA 031-F |
| CD 25 | 3.5% | 8 | 7.3% | 21 | 8.6% | 16 | CD 11a-F |
| | | | | | | | CD 25-F |
| CD 45 RA | 75.5% | 86 | 37.7% | 51 | 33.8% | 43 | CD 45 RA-F |
| CD 45 R0 | 19.0% | 25 | 72.1% | 53 | 70.7% | 38 | CD 45 R0-F |
| CD 71 | 2.9% | 5 | 6.6% | 14 | 8.8% | 12 | CD 71-F |
| HLA-DR | 10.4% | 40 | 19.8% | 45 | 19.7% | 67 | HLA-DR-F |
| Lecam 1 | 24.4% | 14 | 27.2% | 28 | 21.1% | 23 | LECAM 1-F |
| ¹ = Autofluorescence without antibody marking, n.d. = not determined | | | | | | | |

**Table 5: Distribution pattern of different differentiation and proliferation markers on the T helper cells/inflammatory T cells in the respective total population**

| Positive antigen detection | Mononuclear cells, start of culture | | Mononuclear cells, day 34 | | Lymphocytes, day 34 | | Antibodies |
|---|---|---|---|---|---|---|---|
| | Fraction | Median | Fraction | Median | Fraction | Median | |
| Control¹ | 0.1% | 9 | 2.5% | 11 | 5.7% | 11 | |
| TCR ab | 75.4% | 13 | 85.1% | 34 | 85.9% | 29 | BMA 031-F |
| CD 11a | 96.8% | 19 | 98.1% | 55 | 97.3% | 39 | CD 11a-F |
| CD 25 | 3.8% | 10 | 8.4% | 26 | 11.0% | 17 | CD 25-F |
| CD 45 RA | 59.3% | 42 | 23.2% | 38 | 18.8% | 37 | CD 45 RA-F |
| CD 45 R0 | 29.2% | 36 | 78.3% | 61 | 76.9% | 44 | CD 45 R0-F |
| CD 71 | 0.4% | 9 | 3.6% | 15 | 6.5% | 13 | CD 71-F |
| HLA-DR | 3.6% | 7 | 9.9% | 16 | 13.2% | 24 | HLA-DR-F |
| Lecam 1 | 18.6% | 14 | 19.2% | 24 | 18.5% | 26 | LECAM 1-F |
| ¹ = Autofluorescence without antibody marking | | | | | | | |

**Table 6: Distribution pattern of different differentiation and proliferation markers on the T suppressor cells in the respective total population**

| Positive antigen detection | Mononuclear cells, start of culture | | Mononuclear cells, day 34 | | Lymphocytes, day 34 | | Antibodies |
|---|---|---|---|---|---|---|---|
| | Fraction | Median | Fraction | Median | Fraction | Median | |
| Control¹ | 0.1% | 3 | 0.2% | 20 | 1.1% | 5 | |
| TCR ab | 96.8% | 18 | 97.8% | 35 | 96.9% | 31 | BMA 031-F |
| CD 11a | 100.0% | 54 | 100.0% | 93 | 100.0% | 83 | CD 11a-F |
| CD 25 | 2.1% | 8 | 0.4% | 8 | 2.3% | 22 | CD 25-F |
| CD 45 RA | 85.7% | 71 | 61.7% | 51 | 62.0% | 50 | CD 45 RA-F |
| CD 45 R0 | 14.4% | 14 | 64.0% | 31 | 57.7% | 30 | CD 45 R0-F |
| CD 71 | 0.9% | 8 | 0.3% | 45 | 0.4% | 6 | CD 71-F |
| HLA-DR | 6.2% | 9 | 19.1% | 15 | 14.1% | 23 | HLA-DR-F |
| Lecam 1 | 20.3% | 16 | 28.6% | 31 | 21.1% | 23 | LECAM 1-F |
| ¹ = Autofluorescence without antibody marking | | | | | | | |

**Table 7: Detection of different antibodies in the cell culture supernatant of the mentioned populations and in the serum of the MNC donor by ELISA:**

| Antibody detection | Serum of the MNC donor | Culture supernatant of MNC, day 34 | Culture supernatant of MNC, day 56 | Culture supernatant of lymphocytes, day 34 |
|---|---|---|---|---|
| IgM | ++ | + | + | + |
| IgG | +++ | ++ | ++ | ++ |
| Lipid-A antibody | + | -- | -- | -- |
| LPS-J5 antibody | + | -- | -- | -- |
| Anti-HBs | +++ | ++ | + | + |
| antibody | | | | |
| Legend: "--" = 0-0.1; "+" = 0.1-1; "++" = 1-10; "+++" = 10-100 µg/ml | | | | |

**Table 8: Detection of different cytokines in the cell culture supernatant of the mentioned populations**

| Cytokine detection (pg/ml) | Mononuclear cells, day 34 | Mononuclear cells, day 56 | Lymphocytes, day 34 |
|---|---|---|---|
| IL-8 | 3879 | 492 | 4931 |
| IL-1ra | 38020 | 14400 | 36800 |
| IL-1β, IL-2, IL-4, IL-6, TNFα | 0 | 0 | 0 |

### Example 4: Differentiation of leukaemic B lymphocytes into plasma cells applying three dimensional tissue culture

10 ml bone marrow tissue form a patient with B-chronic lymphatic leukaemia (B-CLL) was obtained by biopsy. Over 91% of the nucleated bone marrow cells were CD19/20 positive leukaemic B-lymphocytes, bearing their antibody on the cell surface. A total of 2.57x10⁹ BM-cells with a viability of 100% together with erythrocytes and remaining after biopsy specula's were suspended in 8 ml IFM medium. The cells were filled into the culture space of a Tecnomouse^{®} culture cassette. Tissue re-organization was allowed to take place in that culture room due to effective oxygen supply via two flat silicone membranes and basal media perfusion via hollow fibres. The bioreactor was operated over 145 days at perfusion rates of 50 ml/h. Harvests were taken every two weeks. Aliquots of inoculated and harvested cells and culture medium supernatants of extra capillary space were analysed. Suspended cells were characterized for DC markers by flowcytometry. Supernatants were analysed for secreted immunoglobulins by ELISA and cytokines using the Quantikine-Immunoassay (R&D Systems, Bierman).

In contrast to rapid apoptosis of B-CLL cells *in vitro* described in literature self organisation of the bone marrow tissue in the bioreactor led to *in vivo* like behaviour over at least 14 days. B-cell markers CD19/20 remained on more than 90% of the cells. After that period CD19/20 expression decreases rapidly to finally 10% at day 145. Since day 14 a significant amount of 600 pg/ml IL-6 was accumulated in the three dimensional cell culture space. The Interleukin 6 levels remain very high over the whole culture period as shown in the following table:

| | | | | |
|---|---|---|---|---|
| Day of culture | 14 | 56 | 112 | 145 |
| IL-6 (pg/ml) | 600 | 300 | 450 | 600 |

With the time course of cultivation secreted immunoglobulins accumulated to very high titers. At day 145 1.5 g/I IgM and 3.0 g/I IgG could be found in the harvested supernatant.
Both, lack of CD markers on the lymphocytes at late stage of cultivation as well as large amounts of accumulated IgM and IgG correspond to differentiation of B-CLL tumor cells into non-dividing antibody secreting plasma cells. Driven by cytokines, such as IL6, the microenvironment of patients in vitro bone marrow culture after day 14 subsequently changed to a new plasma cell inducing phenotype. Thus, self-organization and self-reorganization of functional three-dimensional human lymphatic tissue could be induced providing the right bioreactor and process design.

## Claims

1. A device for culturing cells and/or tissue comprising at least one culturing space (2) containing matrix(es) (8) suitable for settling cells and/or tissue and at least two fluid delivering means (14, 16; 27, 38) to deliver different fluid to the space (2) or the matrix (8), whereby at least one cell-free supply liquid or supply gas (4) and at least one mobile cell phase (6) is supplied, wherein
(i) said at least one mobile cell phase (6) flows through the matrix (8) in traverse flow; and
(ii) the preferably horizontal matrix (8) is kept in a hollow chamber (20a, 20b) through which said at least one mobile cell phase (6) flows in traverse flow.

2. The device according to claim 1, wherein the liquid flows (4, 6) can be supplied to the culturing space (2) or the matrix (8) through permeable lines (14, 16), at least one of the lines being permeable to the mobile phase.

3. The device according to claim 1 or 2, wherein said at least one supply liquid (4) can be supplied through permeable lines (14, 16), preferably capillaries, through or along the culturing space (2) or the matrix (8).

4. The device according to claim 1, wherein the administration and removal of cell free supply liquid or gas (4) is provided via microporous membranes.

5. The device according to any one of claims 1 to 5, wherein
(i) the matrix of the culturing space has a porosity which enables sufficient flow-through by the mobile phase, migration of the cells within the matrix and the formation of local flow gradients (microenvironment) and preferably has a porosity of more than 30 µm; and/or
(ii) the hollow spaces of the matrix (8) have diameters of at least double the diameter of the cells of the mobile phase; and/or
(iii) the matrix (8) is selected from gels, porous materials, such as open-pore foams, woven fabrics and non-woven fabrics.

6. The device according to claim 5, wherein the matrix (8) is a sheet, preferably having a thickness of about 1 to 15 mm, more preferably about 2 to 10 mm.

7. The device according to any one of claims 1 to 6, wherein
(i) the liquid flows (4, 6) diffuses or flows through the matrix (8); and/or
(ii) the matrix (8) is supported on one or two sides thereof by support means (18a, 18b) permeable to cells, especially a screen, the pore size of the support means (18a, 18b) preferably being about from 30 to 100 µm; and/or
(iii) a flow of the mobile phase is established by external perfusion fluidics (pumping) or by gravity flow (periodical turning).

8. The device according to any one of claims 1 to 7, wherein said at least one supply liquid or supply gas (4) can be supplied to the matrix (8) through several porous lines (14), especially capillaries, which are preferably running in parallel in the interior of the matrix (8), wherein the diameter of the lines (capillaries) is preferably from 50 to 150 µm, more preferably about 80 to 120 µm, the porosity of the lines (14) (capillaries) is about 30 to 500 kDa, and/or the mutual distance of the lines (14) (capillaries), which are preferably running in parallel, is twice to ten times, preferably three to five times, the capillary diameter.

9. The device according to one or more of claims 1 to 8, wherein
(i) a microenvironment suitable for a tissue-like culture has been formed within the matrix (8) which ensures the formation of concentration gradients and optimum supply of this phase, namely by a continuous or discontinuous directed controllable flow of at least one cell and tissue population through the immobile cell phase present in the matrix (8); and/or
(ii) the mobile cell phase can be recirculated or directly passed through without sedimentation in the remaining circuit; and/or
(iii) antigen can be applied to the culturing space (2) if needed; and/or
(iv) a means is provided with which the harvesting of cells or products can be performed, preferably by means of mechanical, dynamic and/or enzymatic mechanisms.

10. The device according to one or more of claims 1 to 9, which is suitable for performing a method for culturing mammalian cells and/or tissue mimicking cellular structures and immunological functions of immunologically active tissues, said method comprising the step of culturing mammalian cells immobilized in one or more culturing spaces in contact with at least one cell-free supply liquid or a defined gas mix and at least one mobile phase containing cells of a cell type different from that of the immobilized mammalian cells, said cells having regulatory properties or being capable of later forming antibodies.

## Patentansprüche

1. Vorrichtung zum Kultivieren von Zellen und/oder Gewebe, die Folgendes umfasst: wenigstens einen Kulturraum (2), der eine oder mehrere Matrices (8) enthält, die zum Ansiedeln von Zellen und/oder Gewebe geeignet sind, und wenigstens zwei Fluidübertragungsmittel (14, 16; 27, 38) zum Übertragen eines unterschiedlichen Fluids zum Raum (2) oder zur Matrix (8), wodurch wenigstens eine zellfreie Zufuhrflüssigkeit oder ein Zufuhrgas (4) und wenigstens eine mobile Zellphase (6) zugeführt wird, wobei
(i) die wenigstens eine mobile Zellphase (6) in Querströmung durch die Matrix (8) strömt; und
(ii) die vorzugsweise horizontale Matrix (8) in einer hohlen Kammer (20a, 20b) gehalten wird, durch die die wenigstens eine mobile Zellphase (6) in Querströmung strömt.

2. Vorrichtung gemäß Anspruch 1, wobei die Flüssigkeitsströme (4, 6) durch permeable Leitungen (14, 16) dem Kulturraum (2) oder der Matrix (8) zugeführt werden können, wobei wenigstens eine der Leitungen für die mobile Phase permeabel ist.

3. Vorrichtung gemäß Anspruch 1 oder 2, wobei die wenigstens eine Zufuhrflüssigkeit (4) durch permeable Leitungen (14, 16), vorzugsweise Kapillaren, durch oder entlang des Kulturraums (2) oder der Matrix (8) zugeführt werden kann.

4. Vorrichtung gemäß Anspruch 1, wobei die Verabreichung und Entfernung von zellfreier Zufuhrflüssigkeit oder -gas (4) über mikroporöse Membranen erfolgt.

5. Vorrichtung gemäß einem der Ansprüche 1 bis 5, wobei
(i) die Matrix des Kulturraums eine Porosität hat, die ein ausreichendes Durchströmen mit der mobilen Phase, eine Wanderung der Zellen innerhalb der Matrix und die Bildung von lokalen Strömungsgradienten (Mikroumgebung) erlaubt, und vorzugsweise eine Porosität von mehr als 30 µm hat; und/oder
(ii) die Hohlräume der Matrix (8) Durchmesser haben, die wenigstens doppelt so groß sind wie der Durchmesser der Zellen der mobilen Phase; und/oder
(iii) die Matrix (8) aus Gelen, porösen Materialien, wie offenzelligen Schaumstoffen, Geweben und Vliesstoffen ausgewählt ist.

6. Vorrichtung gemäß Anspruch 5, wobei die Matrix (8) ein Flächengebilde ist, das vorzugsweise eine Dicke von etwa 1 bis 15 mm, besonders bevorzugt etwa 2 bis 10 mm, hat.

7. Vorrichtung gemäß einem der Ansprüche 1 bis 6, wobei
(i) die Flüssigkeitsströme (4, 6) durch die Matrix (8) diffundieren oder strömen; und/oder
(ii) die Matrix (8) auf einer oder zwei ihrer Seiten durch Trägermittel (18a, 18b), die für Zellen durchlässig sind, insbesondere ein Sieb, gestützt wird, wobei die Porengröße des Trägermittels (18a, 18b) vorzugsweise etwa 30 bis 100 µm beträgt; und/oder
(iii) eine Strömung der mobilen Phase durch externe Perfusionsfluidik (Pumpen) oder durch Fließen unter der Schwerkraft (periodisches Umdrehen).

8. Vorrichtung gemäß einem der Ansprüche 1 bis 7, wobei die wenigstens eine Zufuhrflüssigkeit oder das Zufuhrgas (4) durch mehrere poröse Leitungen (14), insbesondere Kapillaren, die vorzugsweise parallel im Innern der Matrix (8) verlaufen, der Matrix (8) zugeführt wird, wobei der Durchmesser der Leitungen (Kapillaren) vorzugsweise 50 bis 150 µm, besonders bevorzugt etwa 80 bis 120 µm, beträgt, die Porosität der Leitungen (14) (Kapillaren) etwa 30 bis 500 kDa beträgt und/oder der gegenseitige Abstand der Leitungen (14) (Kapillaren), die.vorzugsweise parallel verlaufen, doppelt bis zehnmal, vorzugsweise drei bis fünfmal, so groß wie der Kapillardurchmesser ist.

9. Vorrichtung gemäß einem oder mehreren der Ansprüche 1 bis 8, wobei
(i) innerhalb der Matrix (8) eine Mikroumgebung gebildet ist, die für eine gewebeähnliche Kultur geeignet ist und die die Bildung von Konzentrationsgradienten und eine optimale Zuführung dieser Phase gewährleistet, nämlich durch eine kontinuierliche oder diskontinuierliche gerichtete steuerbare Strömung von wenigstens einer Zell- und Gewebepopulation durch die immobile Zellphase, die in der Matrix (8) vorhanden ist; und/oder
(ii) die mobile Zellphase in den Kreislauf zurück oder direkt hindurch geführt werden kann, ohne im übrigen Kreislauf zu sedimentieren; und/oder
(iii) gegebenenfalls Antigen auf den Kulturraum (2) angewendet werden kann; und/oder
(iv) ein Mittel bereitgestellt wird, mit dem das Ernten von Zellen oder Produkten durchgeführt werden kann, vorzugsweise mittels mechanischer, dynamischer und/oder enzymatischer Mechanismen.

10. Vorrichtung gemäß einem oder mehreren der Ansprüche 1 bis 9, die geeignet ist, um ein Verfahren zum Kultivieren von Säugerzellen und/oder Gewebe, das zelluläre Strukturen und immunologische Funktionen von immunologisch aktiven Geweben nachbildet, durchzuführen, wobei das Verfahren den Schritt des Kultivierens von Säugerzellen umfasst, die in einem oder mehreren Kulturräumen in Kontakt mit wenigstens einer zellfreien Zufuhrflüssigkeit oder einem definierten Gasgemisch und wenigstens einer mobilen Phase, die Zellen eines Zelltyps enthält, der von demjenigen der immobilisierten Säugerzellen verschieden sind, immobilisiert sind, wobei die Zellen regulatorische Eigenschaften haben oder in der Lage sind, später Antikörper zu bilden.

## Revendications

1. Dispositif pour la culture de cellules et/ou de tissus comprenant au moins un espace de culture (2) renfermant une matrice /des matrices (8) approprié pour le dépôt de cellules et/ou de tissus et au moins deux moyens de fourniture de fluide (14, 16 ; 27, 38) pour délivrer du fluide différent à l'espace (2) ou la matrice (8), de sorte qu'au moins un gaz de fourniture ou un liquide de fourniture exempt de cellules (4) et au moins une phase de cellules mobile (6) sont fournis, dans lequel :
(i) ladite au moins une phase de cellules mobile (6) s'écoule au travers de la matrice (8) dans un écoulement transversal et
(ii) la matrice de préférence horizontale (8) est maintenue dans une chambre creuse (20a, 20b) au travers de laquelle ladite au moins une phase de cellules mobile (6) s'écoule dans un écoulement transversal.

2. Dispositif selon la revendication 1, dans lequel les écoulements de liquide (4, 6) peuvent être fournis à l'espace de culture (2) ou à la matrice (8) par l'intermédiaire de conduites perméables (14, 16), au moins une des conduites étant perméable à la phase mobile.

3. Dispositif selon la revendication 1 ou 2, dans lequel ledit au moins un liquide de fourniture (4) peut être fourni par l'intermédiaire des conduites perméables (14, 16), de préférence des capillaires, au travers ou le long de l'espace de culture (2) ou de la matrice (8).

4. Dispositif selon la revendication 1, dans lequel l'administration et l'élimination du gaz ou du liquide de fourniture exempt de cellules (4) sont obtenues par l'intermédiaire de membranes microporeuses.

5. Dispositif selon l'une quelconque des revendications 1 à 5, dans lequel :
(i) la matrice de l'espace de culture a une porosité qui permet un écoulement traversant suffisant par la phase mobile, une migration des cellules dans la matrice et la formation de gradients d'écoulement locaux (microenvironnement) et, de préférence, a une porosité supérieure à 30 µm et/ou
(ii) les espaces creux de la matrice (8) ont des diamètres au moins le double du diamètre des cellules de la phase mobile et/ou
(iii) la matrice (8) est choisie à partir de gels, de matériaux poreux, comme des mousses à pore ouvert, des tissus tissés et des tissus non tissés.

6. Dispositif selon la revendication 5, dans lequel la matrice (8) est une feuille, de préférence ayant une épaisseur d'environ 1 à 15 mm, mieux encore d'environ 2 à 10 mm.

7. Dispositif selon l'une quelconque des revendications 1 à 6, dans lequel :
(i) les écoulements de liquide (4, 6) diffusent ou s'écoulent au travers de la matrice (8) et/ou
(ii) la matrice (8) est supportée sur un ou deux côtés de celle-ci par des moyens de support (18a, 18b) perméables aux cellules, notamment un écran, la dimension de pore des moyens de support (18a, 18b) étant, de préférence, d'environ 30 à 100 µm et/ou
(iii) un écoulement de la phase mobile est établi par les fluides de perfusion externes (pompage) ou par écoulement par gravité (révolution périodique).

8. Dispositif selon l'une quelconque des revendications 1 à 7, dans lequel ledit au moins un gaz de fourniture ou liquide de fourniture (4) peut être fourni à la matrice (8) au travers de plusieurs conduites poreuses (14), notamment des capillaires qui s'étendent, de préférence, en parallèle à l'intérieur de la matrice (8), dans lequel le diamètre des conduites (capillaires) est, de préférence, de 50 à 150 µm, mieux encore d'environ 80 à 120 µm, la porosité des conduites (14) (capillaires) est d'environ 30 à 500 kDa et/ou la distance mutuelle des conduites (14) (capillaires) qui s'étendent, de préférence, en parallèle est de deux à dix fois, de préférence de trois à cinq fois le diamètre des capillaires.

9. Dispositif selon l'une ou plusieurs des revendications 1 à 8, dans lequel :
(i) un microenvironnement approprié pour une culture analogue à un tissu est formé dans la matrice (8) qui assure la formation de gradients de concentration et la fourniture optimum de cette phase, à savoir par un écoulement contrôlable, continu ou discontinu d'au moins une population de tissus et de cellules au travers de la phase de cellules immobile présente dans la matrice (8) et/ou
(ii) la phase de cellules mobile peut être recyclée ou peut traverser directement sans sédimentation dans le circuit restant et/ou
(iii) un antigène peut être appliqué à l'espace de culture (2) si cela est nécessaire et/ou
(iv) un moyen est prévu dans lequel la récolte des cellules ou des produits peut être réalisée, de préférence au moyen de mécanismes mécaniques, dynamiques et/ou enzymatiques.

10. Dispositif selon l'une ou plusieurs des revendications 1 à 9, qui est approprié pour réaliser un procédé de culture de cellules et/ou de tissus de mammifères imitant les structures cellulaires et les fonctions immunologiques de tissus actifs immunologiquement, ledit procédé comprenant l'étape de culture de cellules de mammifères immobilisées dans un ou plusieurs espaces de culture en contact avec au moins un liquide de fourniture exempt de cellules ou un mélange de gaz défini et au moins une phase mobile contenant des cellules d'un type de cellule différent de celui des cellules de mammifères immobilisées, lesdites cellules ayant des propriétés de régulation ou étant capables de former subséquemment des anticorps.
